# EUROPEAN PATENT APPLICATION

(11) **EP 4 442 305 A1**
(43) Date of publication of application: **09.10.2024**
(21) Application number: 24164384.0
(22) Date of filing: 19.03.2024
(51) Int. Cl.: A61M 29/00, A61F 2/24, A61M 25/00

(54) **PROSTHETIC HEART VALVE DELIVERY ASSEMBLY**

(30) Priority: 24.03.2023 US 202363454450 P; 21.02.2024 US 202418582940
(71) Applicant: Medtronic, Inc., Minneapolis, MN 55432-5604 (US)
(72) Inventor: CLARKE, Luke A., Minneapolis, 55432 (US); CIOBANU, Constantin F., Minneapolis, 55432 (US); MCAFEE, Patricia, Minneapolis, 55432 (US); MORELLI, Fionnuala, Minneapolis, 55432 (US); FARRELL, Timothy D., Minneapolis, 55432 (US); CALLAGY, Colm, Minneapolis, 55432 (US); MORAN, Chris, Minneapolis, 55432 (US)
(74) Representative: Zimmermann & Partner Patentanwälte mbB

(57) **Abstract**

A prosthetic valve delivery assembly includes a dilator including a first tapered region extending along a dilator axis. The first tapered region includes a tapered shape with a first diameter at a distal end and a second diameter at a first central end. The second diameter is greater than the first diameter. A central region includes the second diameter that is substantially constant along a central length of the central region. A second tapered region includes a tapered shape with the second diameter at the second central end and a third diameter at a third central end. The third diameter is less than the second diameter. A proximal shaft region extends from the third central end. The proximal shaft region includes the third diameter such that a difference between the second diameter and the third diameter is within a French gauge range from about 3Fr to about 5Fr.

## Description

### Cross-Reference to Related Applications

This application claims the benefit of U.S. Provisional Patent Application Serial No. 63/454,450, filed March 24, 2023, the entire content of which is incorporated herein by reference.

### FIELD

The present disclosure relates generally to a prosthetic heart valve delivery assembly and, more particularly, to a prosthetic heart valve delivery assembly comprising a dilator with a non-constant diameter.

### BACKGROUND

It is known to provide a prosthetic heart valve assembly for implanting a heart valve prosthesis within a target site of the vasculature of a patient. It is further known to use a dilator and a sheath as part of the implant procedure. However, insertion of the dilator and the sheath within the vasculature can be difficult.

### SUMMARY

The following presents a simplified summary of the disclosure to provide a basic understanding of some aspects described in the detailed description.

In aspects, a prosthetic valve delivery assembly comprises a dilator extending along a dilator axis between a proximal end and a distal end. The dilator comprises a first tapered region extending along the dilator axis between the distal end and a first central end. The first tapered region comprises a tapered shape with a first diameter at the distal end and a second diameter at the first central end. The second diameter is greater than the first diameter. The dilator comprises a central region coaxial with the first tapered region and attached to the first central end. The central region comprises the second diameter that is substantially constant along a central length of the central region. The dilator comprises a second tapered region extending along the dilator axis between a second central end and a third central end. The second central end is attached to the central region. The second tapered region comprises a tapered shape with the second diameter at the second central end and a third diameter at the third central end. The third diameter is less than the second diameter. The dilator comprises a proximal shaft region extending from and in contact with the third central end. The proximal shaft region comprises the third diameter such that a difference between the second diameter and the third diameter is within a French gauge range from about 3Fr to about 5Fr.

In aspects, the central region comprises a central length that is within a range from about 1 mm to about 150 mm.

In aspects, the tapered shape of the second tapered region comprises a taper angle that is within a range from about 1 degree to about 30 degrees.

In aspects, the second diameter is less than about 1.5 times a vessel diameter of the vessel.

In aspects, a handle is attached adjacent to the proximal end of the dilator. The handle is axially fixed relative to the dilator and comprises a non-constant cross-sectional size along a length of the handle.

In aspects, the dilator comprises at least one lumen extending axially through the dilator.

In aspects, the at least one lumen comprises a first lumen, a second lumen, and a third lumen. The first lumen is laterally offset from, and between, the second lumen and the third lumen.

In aspects, the dilator comprises a plurality of materials.

In aspects, a prosthetic valve delivery assembly comprises a sheath comprising a wall surrounding an elongated chamber. The sheath is configured to be received within a vessel. The prosthetic valve delivery assembly comprises a dilator configured to be received within the chamber and extending along a dilator axis between a proximal end and a distal end. The dilator comprises a first tapered region extending along the dilator axis between the distal end and a first central end. The first tapered region comprises a tapered shape with a first diameter at the distal end and a second diameter at the first central end. The second diameter is greater than the first diameter. The dilator comprises a central region coaxial with the first tapered region and attached to the first central end. The central region comprises the second diameter that is less than about 1.5 times a vessel diameter of the vessel. The central region comprises a central length that is within a range from about 1 mm to about 150 mm. The dilator comprises a second tapered region extending along the dilator axis between a second central end and a third central end. The second central end is attached to the central region. The second tapered region comprises a tapered shape with the second diameter at the second central end and a third diameter at the third central end. The third diameter is less than the second diameter. The tapered shape of the second tapered region comprises a taper angle that is within a range from about 1 degree to about 30 degrees. The dilator comprises a proximal shaft region extending from and in contact with the third central end. The proximal shaft region comprises the third diameter. The third diameter is within a range from about 60% to about 90% of the second diameter.

In aspects, a diameter of the central region is non-constant along the central length of the central region, and the second diameter is a maximum diameter of the central region.

In aspects, the central region comprises a first axial location and a second axial location comprising the second diameter. The central region comprises a third axial location positioned between the first axial location and the second axial location and comprising a diameter less than the second diameter.

In aspects, a cross-sectional size of the central region is non-constant about a circumferential perimeter of the central region.

In aspects, the second diameter is constant along the central length of the central region.

In aspects, the central region comprises a material that is different than a material of the proximal shaft region.

In aspects, methods of expanding a sheath comprise positioning a sheath within a vessel. The sheath comprises a wall surrounding an elongated chamber. Methods comprise inserting a dilator within the chamber. The dilator extends along a dilator axis between a proximal end and a distal end. The dilator comprises a first tapered region with an increasing diameter from the distal end, a central region coaxial with and in contact with the first tapered region and comprising a substantially constant diameter, and a second tapered region extending from and in contact with the central region. The second tapered region comprises a tapered shape with a decreasing diameter from the central region. Methods comprise radially expanding the sheath by contacting the wall with the central region.

In aspects, the central region comprises a central length that is within a range from about 1 mm to about 150 mm.

In aspects, methods further comprise moving the dilator axially by applying a force to a handle that is attached adjacent to the proximal end of the dilator. The handle is fixed relative to the dilator and comprises a non-constant cross-sectional size along a length of the handle.

In aspects, methods further comprise receiving a first guidewire within a first lumen that extends axially through the dilator.

In aspects, methods further comprise receiving a second guidewire within a second lumen that extends axially through the dilator, and a third guidewire within a third lumen that extends axially through the dilator. The first lumen is laterally offset from, and between, the second lumen and the third lumen.

In aspects, methods further comprise radially expanding the vessel by contacting a vessel wall of the vessel with the central region.

Additional features and advantages of the aspects disclosed herein will be set forth in the detailed description that follows, and in part will be clear to those skilled in the art from that description or recognized by practicing the aspects described herein, including the detailed description which follows, the claims, as well as the appended drawings. It is to be understood that both the foregoing general description and the following detailed description present aspects intended to provide an overview or framework for understanding the nature and character of the aspects disclosed herein. The accompanying drawings are included to provide further understanding and are incorporated into and constitute a part of this specification. The drawings illustrate various aspects of the disclosure, and together with the description explain the principles and operations thereof.

### BRIEF DESCRIPTION OF THE DRAWINGS

These and other features, aspects and advantages are better understood when the following detailed description is read with reference to the accompanying drawings, in which:
**FIG. 1** schematically illustrates example aspects of a transcatheter heart valve prosthesis in accordance with aspects of the disclosure;
**FIG. 2** illustrates a top-down view of the transcatheter heart valve prosthesis in accordance with aspects of the disclosure;
**FIG. 3** illustrates a side view of a delivery assembly for delivering the transcatheter heart valve prosthesis in accordance with aspects of the disclosure;
**FIG. 4** illustrates a side view of the delivery assembly for delivering the transcatheter heart valve prosthesis in accordance with aspects of the disclosure;
**FIG. 5** illustrates an introducer sheath in accordance with aspects of the disclosure;
**FIG. 6** illustrates an introducer sheath in accordance with aspects of the disclosure;
**FIG. 7** schematically illustrates a side view of a dilator and a sheath in accordance with aspects of the disclosure;
**FIG. 8** illustrates a perspective view of an end of the dilator in accordance with aspects of the disclosure;
**FIG. 9** schematically illustrates a side view of a handle attached to the dilator in accordance with aspects of the disclosure;
**FIG. 10** schematically illustrates a side view of the dilator and the sheath positioned in a vessel in accordance with aspects of the disclosure;
**FIG. 11** schematically illustrates a side view of the dilator positioned in a vessel in accordance with aspects of the disclosure
**FIG. 12** illustrates additional aspects of a dilator in accordance with aspects of the disclosure;
**FIG. 13** illustrates additional aspects of a dilator in accordance with aspects of the disclosure;
**FIG. 14** illustrates additional aspects of a dilator in accordance with aspects of the disclosure;
**FIG. 15** illustrates additional aspects of a dilator in accordance with aspects of the disclosure;
**FIG. 16** illustrates additional aspects of a dilator in accordance with aspects of the disclosure;
**FIG. 17** illustrates a cross-sectional view of the dilator of **FIG. 16** along lines **16-16** of **FIG. 16** in accordance with aspects of the disclosure
**FIG. 18** illustrates additional aspects of a dilator in accordance with aspects of the disclosure; and
**FIG. 19** illustrates additional aspects of a dilator in accordance with aspects of the disclosure.

### DETAILED DESCRIPTION

Aspects will now be described more fully hereinafter with reference to the accompanying drawings in which example aspects are shown. Whenever possible, the same reference numerals are used throughout the drawings to refer to the same or like parts. However, this disclosure may be embodied in many different forms and should not be construed as limited to the aspects set forth herein.

As used herein, the term "about" means that amounts, sizes, formulations, parameters, and other quantities and characteristics are not, and need not be, exact, but may be approximate and/or larger or smaller, as desired, reflecting tolerances, conversion factors, rounding off, measurement error and the like, and other factors known to those of skill in the art.

Ranges can be expressed herein as from "about" one value, and/or to "about" another value. When such a range is expressed, aspects include from the one value to the other value. Similarly, when values are expressed as approximations by use of the antecedent "about," it will be understood that the value forms another aspect. It will be further understood that the endpoints of each of the ranges are significant both in relation to the other endpoint, and independently of the other endpoint.

Directional terms as used herein - for example up, down, right, left, front, back, top, bottom, upper, lower, etc. - are made only with reference to the figures as drawn and are not intended to imply absolute orientation.

Unless otherwise expressly stated, it is in no way intended that any methods set forth herein be construed as requiring that its steps be performed in a specific order, nor that with any apparatus, specific orientations be required. Accordingly, where a method claim does not actually recite an order to be followed by its steps, or that any apparatus claim does not actually recite an order or orientation to individual components, or it is not otherwise specifically stated in the claims or description that the steps are to be limited to a specific order, or that a specific order or orientation to components of an apparatus is not recited, it is in no way intended that an order or orientation be inferred in any respect. This holds for any possible non-express basis for interpretation, including matters of logic relative to arrangement of steps, operational flow, order of components, or orientation of components; plain meaning derived from grammatical organization or punctuation, and; the number or type of aspects described in the specification.

As used herein, the singular forms "a," "an," and "the" include plural references unless the context clearly dictates otherwise. Thus, for example, reference to "a" component includes aspects having two or more such components, unless the context clearly indicates otherwise.

The word "exemplary," "example," or various forms thereof are used herein to mean serving as an example, instance, or illustration. Any aspect or design described herein as "exemplary" or as an "example" should not be construed as preferred or advantageous over other aspects or designs. Furthermore, examples are provided solely for purposes of clarity and understanding and are not meant to limit or restrict the disclosed subject matter or relevant portions of this disclosure in any manner. It can be appreciated that a myriad of additional or alternate examples of varying scope could have been presented but have been omitted for purposes of brevity.

As used herein, the terms "comprising," "including," and variations thereof shall be construed as synonymous and open-ended, unless otherwise indicated. A list of elements following the transitional phrases comprising or including is a non-exclusive list, such that elements in addition to those specifically recited in the list may also be present.

The terms "substantial," "substantially," and variations thereof as used herein are intended to represent that a described feature is equal or approximately equal to a value or description. For example, a "substantially planar" surface is intended to denote a surface that is planar or approximately planar. Moreover, "substantially" is intended to denote that two values are equal or approximately equal. The term "substantially" may denote values within about 10% of each other, for example, within about 5% of each other, or within about 2% of each other.

Modifications may be made to the instant disclosure without departing from the scope or spirit of the claimed subject matter. Unless specified otherwise, "first," "second," or the like are not intended to imply a temporal aspect, a spatial aspect, an ordering, etc. Rather, such terms are merely used as identifiers, names, etc. for features, elements, items, etc. For example, a first end and a second end generally correspond to end A and end B or two different ends.

Unless otherwise indicated, the terms "distal" and "proximal" are used in the following description with respect to a position or direction relative to the treating clinician. "Distal" and "distally" are positions distant from or in a direction away from the clinician, and "proximal" and "proximally" are positions near or in a direction toward the clinician.

In addition, the term "self-expanding" may be used in the following description with reference to one or more valve or stent structures of the prostheses hereof and is intended to convey that the structures are shaped or formed from a material that can be provided with a mechanical memory to return the structure from a compressed or constricted delivery configuration to an expanded deployed configuration or vice versa. Non-exhaustive exemplary self-expanding materials include stainless steel, a pseudoelastic metal such as a nickel titanium alloy or nitinol, various polymers, or a so-called super alloy, which may have a base metal of nickel, cobalt, chromium, or other metal. Mechanical memory may be imparted to a wire or stent structure by thermal treatment to achieve a spring temper in stainless steel, for example, or to set a shape memory in a susceptible metal alloy, such as nitinol. Various polymers that can be made to have shape memory characteristics may also be suitable for use in aspects hereof to include polymers such as polynorborene, trans-polyisoprene, styrene-butadiene, and polyurethane. As well poly L-D lactic copolymer, oligo caprylactone copolymer and poly cyclo-octine can be used separately or in conjunction with other shape memory polymers.

Diseases associated with heart valves, such as those caused by damage or a defect, can include stenosis and valvular insufficiency or regurgitation. For example, valvular stenosis causes the valve to become narrowed and hardened which can prevent blood flow to a downstream heart chamber from occurring at the proper flow rate and may cause the heart to work harder to pump the blood through the diseased valve. Valvular insufficiency or regurgitation occurs when the valve does not close completely, allowing blood to flow backwards, thereby causing the heart to be less efficient. A diseased or damaged valve, which can be congenital, age-related, drug-induced, or in some instances, caused by infection, can result in an enlarged, thickened heart that loses elasticity and efficiency. Some symptoms of heart valve diseases can include weakness, shortness of breath, dizziness, fainting, palpitations, anemia and edema, and blood clots which can increase the likelihood of stroke or pulmonary embolism. Symptoms can often be severe enough to be debilitating and/or life threatening.

Heart valve prostheses have been developed for repair and replacement of diseased and/or damaged heart valves. Such heart valve prostheses can be percutaneously delivered and deployed at the site of the diseased heart valve through catheter-based delivery systems. Such heart valve prostheses generally include a frame or stent and a prosthetic valve mounted within the frame. Such heart valve prostheses are delivered in a radially compressed or crimped configuration so that the heart valve prosthesis can be advanced through the patient's vasculature. Once positioned at the treatment site, the heart valve prosthesis is expanded to engage tissue at the diseased heart valve region to, for instance, hold the heart valve prosthesis in position.

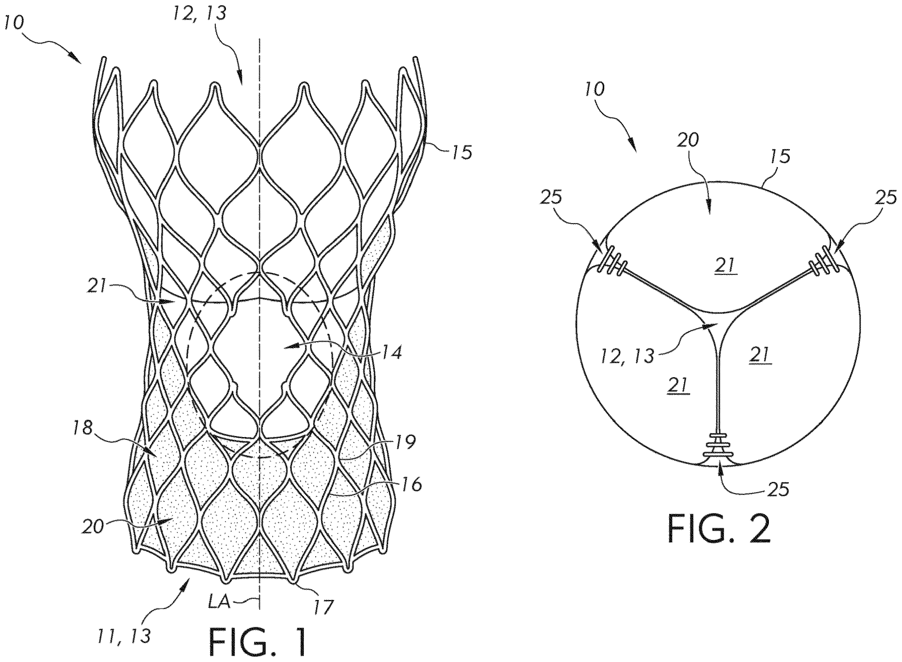

**FIGS. 1** and **2** illustrate an example transcatheter heart valve prosthesis **10.** The delivery assemblies described herein may be used with the transcatheter heart valve prosthesis **10** and/or other transcatheter heart valve prostheses. The transcatheter heart valve prosthesis **10** is illustrated to facilitate description of the disclosure. The following description of the transcatheter heart valve prosthesis **10** is merely exemplary in nature and is not intended to limit the invention or the application and uses of the invention.

**FIGS. 1** and **2** illustrate a side view and a top (outflow end) view, respectively, of the transcatheter heart valve prosthesis **10.** The transcatheter heart valve prosthesis **10** includes a radially-expandable frame or stent **15** and a prosthetic valve **20.** The frame **15** of the transcatheter heart valve prosthesis **10** supports the prosthetic valve **20** within an interior of the frame **15.** In the example transcatheter heart valve prosthesis **10** shown in **FIGS. 1** and **2****,** the frame **15** is self-expandable. However, this is not meant to be limiting, and the frame **15** can be balloon-expandable or mechanically expandable in other embodiments.

The prosthetic valve **20** includes at least one leaflet **21** disposed within and secured to the frame **15.** In the embodiment shown in **FIGS. 1** and **2****,** the prosthetic valve **20** includes exactly three leaflets **21,** as shown in **FIG. 2****.** However, this is not meant to be limiting, as the prosthetic valve **20** may include more or fewer leaflets **21.** The valve leaflets **21** open and close to regulate flow through the transcatheter heart valve prosthesis **10.**

As shown in **FIG. 1****,** the transcatheter heart valve prosthesis **10** includes an inflow end **11** and an outflow end **12.** The prosthetic leaflets **21** are attached to the frame **15** at commissures **25** such that when pressure at the inflow end **11** exceeds pressure at the outflow end **12,** the prosthetic leaflets **21** open to allow blood flow through the heart valve prosthesis **10** from the inflow end **11** to the outflow end **12.** When the pressure at the outflow end **12** exceeds pressure at the inflow end **11,** the prosthetic leaflets **21** close to prevent blood flow from the outflow end **12** to the inflow end **11.** Accordingly, the at least one leaflet (e.g., the prosthetic leaflets **21**) can be attached to the plurality of struts **16,** for example, by being directly attached to the plurality of struts **16** at the commissures **25,** or by being indirectly attached to the plurality of struts **16,** for example, by being attached to a skirt, a commissure bracket, or other structure (e.g., mechanical actuator) that is attached to the plurality of struts **16.**

The frame **15** of the transcatheter heart valve prosthesis **10** further includes a plurality of struts **16** that are arranged to form a plurality of openings or cells **18** arranged circumferentially around a longitudinal axis LA of the transcatheter heart valve prosthesis **10** and longitudinally to form a tubular structure defining a central lumen **13** of the transcatheter heart valve prosthesis **10.** For example, the frame **15** can extend along the longitudinal axis LA between the inflow end **11** and the outflow end **12.** The frame **15** is configured to secure the prosthetic valve **20** within the central lumen **13** of the frame **15** and to secure the transcatheter heart valve prosthesis **10** in place in the vasculature of the patient. The struts **16** are defined herein as the elongated wire segments of the frame **15.** Struts **16** come together to form crowns **17** or nodes **19,** as can be seen in **FIG. 1****.** The frame **15** of the heart valve prosthesis **10** includes a plurality of cells **18** defined as the spaces between the plurality of crowns **17,** the plurality of nodes **19,** and the plurality of struts **16.** The frame **15,** and, thus, the plurality of struts **16,** can be adjustable between a radially-collapsed position and a radially-expanded position.

In the example embodiment shown in **FIG. 1****,** the plurality of cells **18** may be diamond-shaped. In the example embodiment shown, the plurality of cells include a plurality of first cells **18** and access cells **14.** In particular, the access cells are larger than the first cells **18** and can provide access to one or more coronary arteries when the transcatheter heart valve prosthesis **10** is implanted in the patient. In the embodiment shown, there are exactly three access cells **14.** However, this is not meant to be limiting, as the frame **15** of the transcatheter heart valve prosthesis **10** can include more, fewer, or no access cells **14.** The access cells **14** each have an enlarged area relative or compared to the first cells **18,** as can be seen in **FIG. 1****.** Further, the access cells **14** may be located in other locations than the locations shown in **FIG. 1****.** Although not shown, in some embodiments the transcatheter heart valve prosthesis **10** may include an outer skirt extending circumferentially around an outer circumference of the stent **15** at or near the inflow end **11** to prevent paravalvular leakage of blood around the outside of the transcatheter heart valve prosthesis **10** once implanted in the patient.

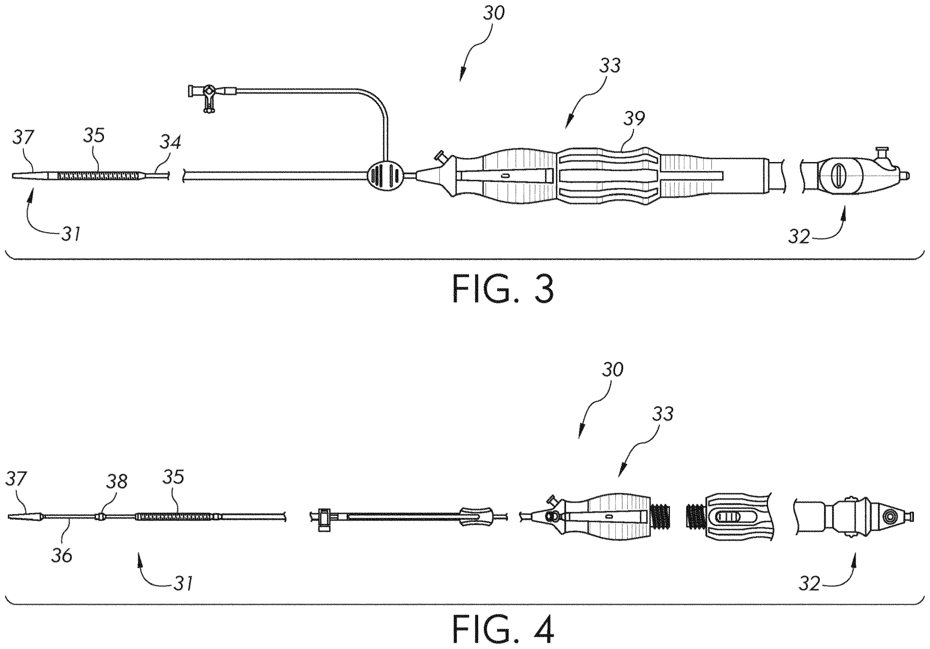

**FIGS. 3** and **4** show schematically side views of a delivery assembly **30** for delivering and deploying a transcatheter heart valve prosthesis (e.g., transcatheter heart valve prosthesis **10**) according to embodiments hereof. One skilled in the art will realize that **FIGS. 3** and **4** illustrate one example of a delivery assembly **30** and that components illustrated in **FIGS. 3** and **4** may be removed and/or additional components may be added. The delivery assembly **30** includes a distal end **31,** a proximal end **32,** and a handle **33.** The handle **33** enables a physician to manipulate a distal portion of the delivery assembly **30** and includes actuators for moving parts of the delivery assembly **30** relative to other parts. In the delivery assembly **30,** an outer shaft **34** is coupled to an actuator **39** of the handle **33** for moving the outer shaft **34** relative to an inner shaft **36.**

A distal portion of the outer shaft **34,** referred to as a capsule **35,** is configured to surround a transcatheter heart valve prosthesis (e.g., transcatheter heart valve prosthesis **10**) during delivery to the treatment site (e.g., a native heart valve) and is retracted from the transcatheter heart valve prosthesis to expose the transcatheter heart valve prosthesis such that it self-expands. The inner shaft **36** is coupled to the handle **33** and movement of the handle **33** translates to movement of the inner shaft **36** and a distal tip or nosecone **37** coupled to a distal end of the inner shaft **36.** The inner shaft **36** and distal tip or nosecone **37** may also be translated relative to the outer shaft **34** and the handle **33** via a tip retractor. In the embodiment shown, the inner shaft **36** includes a retainer or spindle **38** for receiving the paddles of the transcatheter heart valve prosthesis **10.**

When the actuator **39** is actuated, the actuator **39** moves the outer shaft **34** and the capsule **35** relative to the inner shaft **36,** as shown in **FIG. 4****.** As known to those skilled in the art, when the delivery assembly **30** is in position such that the transcatheter heart valve prosthesis **10** is at the desired position at the treatment site in the patient's vasculature, the actuator **39** is actuated to move the capsule **35** relative to the inner shaft **36** and the transcatheter heart valve prosthesis **10** disposed between the inner shaft **36** and the capsule **35,** thereby enabling the transcatheter heart valve prosthesis **10** to deploy via self-expansion at the treatment site and release from the retainer **38,** as shown in **FIG. 4** (without showing the transcatheter heart valve prosthesis **10**).

Minimally invasive percutaneous interventional procedures, including endovascular procedures, require access to the venous or arterial system. In general, it is desirable to make the smallest incision point with the shortest tissue contact time when entering the body. Small incisions and short tissue contact time generally lead to improved patient outcomes, less complications, and less trauma to the vessels or organs being accessed, as well as less trauma to the skin and tissue through which the access point is created. Access is required for various medical procedures that deliver or implant structural elements (such as heart valves, heart valve repair devices, occluders, grafts, electrical stimulators, leads, etc.) percutaneously. Some procedures employ relatively large devices that require relatively large sheaths to deliver the devices to the intended site within the body. With such procedures, access site trauma can occur, often resulting in vessel damage, excessive bleeding, increased case time, increased risk of infection, and increased hospitalization time. To reduce access trauma, physicians try to use the smallest devices possible and place the smallest sheath size. This can be problematic, however, if during the procedure the physician discovers a larger device is needed. This leads to a need to upsize the sheath, which is a lengthy procedure and leads to increased risk to the patient. Expandable sheaths can be expanded within the body and thus do not require removal to upsize.

Expandable sheath designs may be regionally or locally expansive to selectively and temporarily expand when the device is passing through a region of the sheath and to retract or recover when the device is not passing or has already passed through the sheath. Embodiments disclosed herein may be employed with an expandable introducer sheath that may solve these and other issues that contribute to vascular trauma. The expandable introducer sheath disclosed herein is described with respect to percutaneous access for transcatheter heart valve repair or replacement, and it should be understood that one or more features of the expandable introducer sheath may be employed alone or in combination for other medical procedures requiring percutaneous access, including but not limited to placement of stents, angioplasty, removal of arterial or venous calcification, and pre-dilatation or post-dilatation.

Various embodiments disclosed herein may include an introducer sheath that has a selectively expandable diameter to allow for the passage of a relatively larger device therethrough and further is configured to return to its original diameter upon passage of the device. The various embodiments may reduce damage to surrounding tissues by reducing contact with those tissues and by eliminating the need to exchange sheaths of different sizes. As a result, in comparison to known sheaths, these embodiments can reduce procedure time, vascular trauma, bleeding, and the resulting risk of infection and other complications. However, it should be understood that the present disclosure is not limited for use with an expandable introducer sheath. Rather, one or more features of the present disclosure can be employed either alone or in combination without an introducer sheath, with a non-expandable introducer sheath, or with an expandable introducer sheath. Likewise, if employed, the introducer sheath may be an integrated introducer sheath (e.g., an introducer sheath integrated with a delivery assembly) or a non-integrated introducer sheath (e.g., an introducer sheath separate from the delivery assembly but provided for use with the delivery assembly).

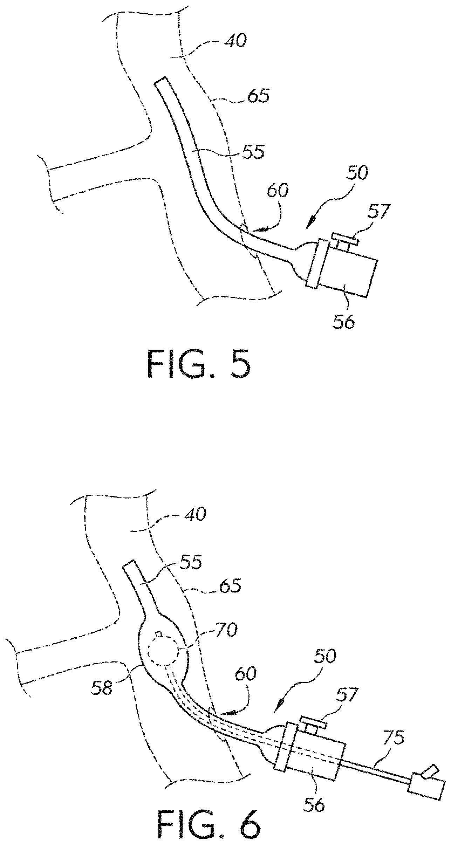

**FIGS. 5** and **6** depict one embodiment of an introducer sheath **50** positioned through an incision **60** in the skin **65** of a patient and into a vessel **40** of a patient. The sheath **50** has a tubular shaft **55** and a proximal hub **56** with a hemostatic seal and a luer lock **57.** **FIG. 5** shows the sheath **50** positioned in the vessel **40** in its normal, unexpanded state, while **FIG. 6** shows the sheath **50** positioned in the vessel **40** with a delivery device **75** delivering another device **70** that is being advanced through the sheath **50** such that the tubular shaft **55** expands or deforms at the location where the device **70** is passing through. The shaft **55** expands at expanded region **58** when the device **70** passes through and then retracts or recovers to its original diameter after the device **70** moves past or is removed from the shaft **55.** Thus, the tubular shaft **55** is configured to be expandable and retractable.

In certain embodiments, the expandability of the shaft **55** (and any shaft described according to any embodiment set forth herein) is achieved via the elasticity of the shaft **55,** which can result in the shaft **55** being either self-expandable or self-expanding or mechanically expandable or mechanically expanding. For purposes of this application, self-expandable means that the shaft **55** is configured to expand to a predetermined or nominal diameter automatically (without any type of actuation, mechanical or otherwise). Further, for purposes of this application, mechanically expandable means that the shaft **55** is configured to expand when a positionable medical device is positioned through the shaft **55.** That is, the device itself that is being passed through the shaft **55** causes the expansion of the shaft **55,** as depicted in **FIG. 6****.** Alternatively, the expandable characteristics of the shaft **55** can be caused by something other than elasticity.

After passage of the device, the shaft **55** is configured to be contractable, retractable, or recoverable to its original, unexpanded state as depicted in **FIG. 5****.** The retractability can be, in certain embodiments, achieved by the elasticity of the shaft **55,** which can result in the shaft **55** being either self-retractable or self-retracting, selfrecoverable, or self-contractable, or mechanically retractable or mechanically retracting, mechanically recoverable, or mechanically contractable. For purposes of this application, self-retractable means that the shaft **55** is configured to retract to a predetermined or nominal diameter automatically (without any type of actuation, mechanical or otherwise). Further, for purposes of this application, mechanically retractable means that the shaft **55** is configured to retract when a device or component is used to cause the shaft **55** to retract or recover. Alternatively, the retractable characteristics of the shaft **55** can be caused by something other than elasticity.

For purposes of this application, any device that can be positioned through an introducer sheath according to any embodiment disclosed or contemplated herein can be referred to as a positionable medical device or insertable medical device. Such devices include guidewires, dilators, delivery devices (for delivery and/or placement of structural elements such as heart valves, heart valve repair devices, occluders, grafts, electrical stimulators, leads, etc.), guide catheters, guiding sheaths, diagnostic catheters, stent delivery systems, balloon catheters, and other known vascular devices. Other devices can include non-vascular devices such as scopes and other common surgical instruments. Further, the introducer sheath is configured to receive tissues or organs. Thus, as one nonlimiting example, the introducer sheath **50** is described as being an expandable introducer sheath **50** for introduction of a delivery assembly **30** including a transcatheter heart valve prosthesis **10.**

As discussed more fully below, dilators and introducer sheaths (e.g., introducer sheath **50**) may be provided as a pair of components that can be included in a vascular introducer set. An introducer set may contain devices used to access blood vessels (e.g., vessel **40**) for the insertion of vascular catheters (e.g., delivery assembly **30**). For example, after a needle is inserted through the skin **65** at incision **60** and into the blood vessel **40,** a dilator and sheath **50** are passed together into the blood vessel **40.** The tapered tip of the dilator acts to stretch the opening **60** in the skin **65** and the blood vessel **40** to allow for the insertion of the introducer sheath **50.** The dilator may also be employed to dilate the introducer sheath **50** within the vessel **40.** The dilator is then removed, leaving only the introducer sheath **50** inserted into the blood vessel **40,** providing an access port through which a variety of catheters (e.g., delivery assembly **30**) can be inserted.

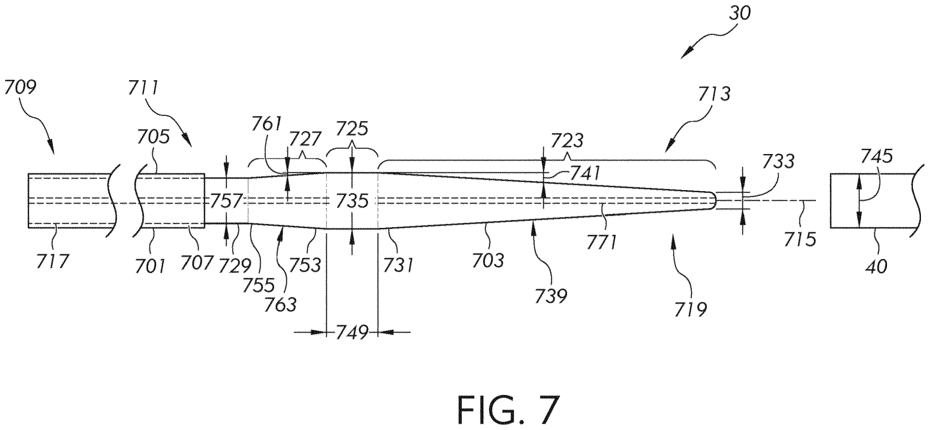

**FIG. 7** illustrates a side view of a dilator **703** in accordance with aspects of the disclosure. The dilator **703** can be employed with a sheath **701** (e.g., introducer sheath **50**) either alone or in combination with delivery assembly **30.** The dilator **703** can be received within the vessel **40** to dilate the vessel **40.** For purposes of illustration, the sheath **701** and the dilator **703** are illustrated as being outside of the vessel **40** in **FIG. 7****.** However, during the process of delivering the valve prosthesis **10,** the sheath **701** and the dilator **703** can be positioned within the vessel **40.** The sheath **701** comprises a wall **705** surrounding an elongated chamber **707.** The sheath **701** can extend between a proximal end **709** and a distal end **711,** and, in aspects, the proximal end **709** may be attached to, or in operative association with, the proximal hub **56.** In aspects, the sheath **701** is received within the vessel **40** and can be accessed through the proximal hub **56.** The dilator **703** can be received within the chamber **707.** The dilator **703** can extend along a dilator axis **715** between a proximal end **717** and a distal end **719.**

The dilator **703** can comprise a tapered end portion **713** comprising one or more regions, for example, a first tapered region **723,** a central region **725,** a second tapered region **727,** and a proximal shaft region **729.** With regard to the first tapered region **723,** the first tapered region **723** can extend along the dilator axis **715** between the distal end of the dilator **703** and a first central end **731.** The first tapered region **723** can comprise a length (e.g., distance between the distal end **719** and the first central end **731**) that may be within a range from about 10 millimeters ("mm") to about 100 mm. In aspects, the first tapered region **723** can comprise a tapered shape with a first diameter **733** at the distal end **719** and a second diameter **735** at the first central end **731,** with the second diameter **735** greater than the first diameter **733.** The tapered shape can comprise a decreasing cross-sectional size (e.g., diameter) from the first central end **731** to the distal end **719.** In aspects, the first tapered region **723** can comprise a constant tapered shape that decreases in diameter at a constant rate from the first central end **731** to the distal end **719.** In aspects, an outer radial surface **739** of the first tapered region **723** can form an angle **741** relative to an axis that is parallel to the dilator axis **715,** with the angle **741** in a range from about 1 degree to about 5 degrees, or from about 2 degrees to about 3 degrees, or about 2.5 degrees. The first tapered region **723** can reduce the likelihood of damage to the vessel **40** when the dilator **703** is inserted into, and moves relative to, the vessel **40.**

The central region **725** can be coaxial with the first tapered region **723** and may be attached to the first central end **731** of the first tapered region **723.** In this way, the central region **725** and the first tapered region **723** can be continuous, such that the first tapered region **723** abuts the central region **725** at the first central end **731.** The central region **725** can comprise the second diameter **735,** such that the central region **725** can comprise the same cross-sectional size as the first central end **731** of the first tapered region **723.** In aspects, the second diameter **735** can be less than about 1.5 times a vessel diameter **745** of the vessel **40,** wherein, in aspects, the vessel diameter **745** may comprise the minimum vessel size of the vessel **40** in the track path of the vasculature. For example, in aspects, the vessel **40** may comprise a femoral artery that may comprise a diameter of about 6 mm, such that the dilator **703** may be selected such that the second diameter **735** is less than about 9 mm. In this way, in aspects, the cross-sectional size of the central region **725** may be greater than the cross-sectional size of the vessel **40.** In aspects, the second diameter **735** can be less than a diameter of the valve prosthesis **10** when the valve prosthesis **10** is in the radially-compressed or crimped configuration, for example, with the second diameter **735** within a range from about 90% to about 95% of a diameter of the valve prosthesis **10** when the valve prosthesis **10** is in the radially-compressed or crimped configuration. In aspects, the second diameter **735** may be substantially constant along a central length **749** of the central region **725.** For example, the central region **725** can comprise the central length **749** that may be within a range from about 1 mm to about 150 mm, or about 5 mm to about 30 mm, or about 10 mm to about 25 mm. In this way, the central region **725** can comprise a cylindrical shape with a substantially constant cross-sectional size (e.g., the second diameter **735**) between the first tapered region **723** and the second tapered region **727.** The central length **749** of the central region **725** can be selected to expand the sheath **701** and/or the vessel **70** for a long enough length to ensure that dilation has occurred.

The second tapered region **727** can be coaxial with the first tapered region **723** and the central region **725.** The second tapered region **727** can extend along the dilator axis **715** between a second central end **753** and a third central end **755,** with the second central end **753** attached to the central region **725.** In this way, the central region **725** and the second tapered region **727** can be continuous, such that the central region **725** abuts the second tapered region **727** at the second central end **753.** Accordingly, the central region **725** can extend between opposing ends, with a distal end attached to the first tapered region **723** and an opposing proximal end attached to the second tapered region **727.** In aspects, the second tapered region **727** can comprise a tapered shape with the second diameter **735** at the second central end **753** and a third diameter **757** at the third central end **755.** The third diameter **757** may be less than the second diameter **735.** In this way, the tapered shape can comprise a decreasing cross-sectional size (e.g., diameter) from the second central end **753** to the third central end **755.** In aspects, the second tapered region **727** can comprise a constant tapered shape that decreases in diameter at a constant rate from the second central end **753** to the third central end **755.** The second tapered region **727** can comprise a length (e.g., distance between the second central end **753** and the third central end **755**) that may be within a range from about 5 mm to about 100 mm. In aspects, a length of the second tapered region **727** may be less than a length of the first tapered region **723** and/or less than the central length **749** of the central region **725.** The tapered shape of the second tapered region **727** can comprise a taper angle **761** that is within a range from about 1 degree to about 30 degrees, or from about 1 degree to about 10 degrees, or from about 2 degrees to about 6 degrees. The taper angle **761** can be measured between an outer radial surface **763** of the second tapered region **727** and an axis that is parallel to the dilator axis **715.** The taper angle **761** can be selected to ensure a smooth transition between the varying diameters, for example, a smooth transition between the central region **725** and the second tapered region **727** and a smooth transition between the second tapered region **727** and the proximal shaft region **729.** Further, by being less than about 30 degrees, the taper angle **761** can be selected to reduce the likelihood of a kink or other sharp twist or bend at a location between the second tapered region **727** and the proximal shaft region **729.**

The proximal shaft region **729** can extend from, and may be in contact with, the third central end **755.** For example, the proximal shaft region **729** can be attached to the third central end **755** of the second tapered region **727** such that the proximal shaft region **729** and the second tapered region **727** can be continuous, such that the proximal shaft region **729** abuts the second tapered region **727** at the third central end **755.** The proximal shaft region **729** can comprise the third diameter **757** that may be within a range from about 60% to about 90% of the second diameter **735.** In this way, the proximal shaft region **729** may comprise a cross-sectional size (e.g., third diameter **757**) that is less than a cross-sectional size (e.g., second diameter **735**) of the central region **725**. In aspects, a difference between the second diameter **735** and the third diameter **757** may be within a French gauge range from about 3Fr (e.g., corresponding to about 1 mm or a circumference of about 3.14 mm) to about 5Fr (e.g., corresponding to about 1.67 mm or a circumference of about 5.24 mm), or about 4Fr. In aspects, the third diameter 757 can be within a range from about 65% to about 95% of the second diameter 735. The proximal shaft region 729 can extend between the proximal end 717 of the dilator 703 and the third central end 755 of the second tapered region 727 with the substantially constant third diameter 757 along the length of the proximal shaft region 729. The cross-sectional size difference between the proximal shaft region **729** and the central region **725** can allow for a balance between the flexural rigidity and the axial rigidity of the dilator **703.** For example, if the diameter of the proximal shaft region **729** is too small, then the proximal shaft region **729** may not comprise sufficient pushability (e.g., axial rigidity) to expand the sheath **701** and the vessel **40.** If the diameter of the proximal shaft region **729** is too large, then the proximal shaft region **729** may be difficult to move the dilator **703** through the vessel **40** due to higher flexural rigidity and contact surface area of the dilator **703.**

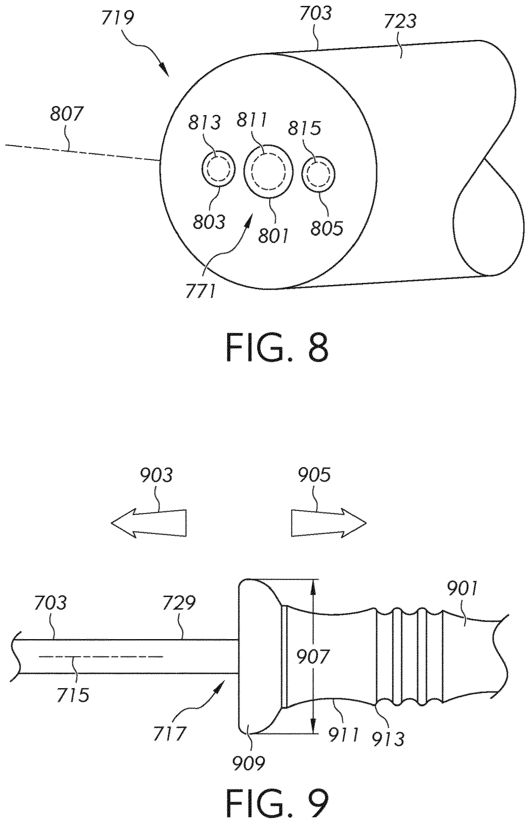

The dilator **703** can comprise at least one lumen **771** extending through the dilator **703** parallel to the dilator axis **715** between the proximal end **717** and the distal end **719.** The at least one lumen **771** may be substantially hollow such that at least one guidewire can be received within the at least one lumen **771.** In aspects, the regions 723, **725, 727** may be closed in a radial direction and devoid of openings extending in the radial direction such that the regions **723, 725, 727** may circumferentially surround the at least one lumen **771.** **FIG. 8** illustrates a perspective view of the distal end **719** of the dilator **703.** In aspects, the at least one lumen **771** can comprise a plurality of lumen, for example, a first lumen **801,** a second lumen **803,** and a third lumen **805.** The first lumen **801** can be laterally offset from, and between, the second lumen **803** and the third lumen **805.** For example, a radial axis **807** can intersect the first lumen **801,** the second lumen **803,** and the third lumen **805** (e.g., while extending in a radial direction through the dilator **703**), with the axis **807** substantially perpendicular to the dilator axis **715** along which the first lumen **801** can extend. In this way, the first lumen **801** may be positioned at a center of the dilator **703,** with the second lumen **803** on a first side of the first lumen **801** and the third lumen **805** on an opposing second side of the first lumen **801.**

In aspects, each of the lumen **801, 803, 805** can receive a guidewire. For example, the first lumen **801** can receive a first guidewire **811,** the second lumen **803** can receive a second guidewire **813,** and the third lumen **805** can receive a third guidewire **815.** The guidewires **811, 813, 815** can exit the dilator **703** at the distal end **719** and may extend along a length of the dilator **703** to the proximal end **717.** The guidewires **811, 813, 815** are illustrated with dashed lines for illustrative purposes and to not obstruct a view of the lumen **801, 803, 805.** In aspects, the dilator **703** can move relative to the guidewires **811, 813, 815.** In this way, methods of expanding the sheath **701** can comprise receiving the first guidewire **811** within the first lumen **801** that extends axially through the dilator **703.** Methods can further comprise receiving the second guidewire **813** within the second lumen **803** that extends axially through the dilator **703,** and receiving the third guidewire **815** within the third lumen **805** that extends axially through the dilator **703,** with the first lumen **801** laterally offset from, and between, the second lumen **803** and the third lumen **805.** While **FIG. 8** illustrates the at least one lumen **771** comprising three lumen **801, 803, 805,** the dilator **703** is not so limited. Rather, in aspects, the at least one lumen **771** can comprise a single lumen, for example, the first lumen **801,** that can receive the first guidewire **811** such that the first lumen **801** and the first guidewire **811** can extend substantially coaxially along the dilator axis **715.**

**FIG. 9** illustrates a side view of the proximal end **717** of the dilator **703,** wherein the dilator **703** can comprise a handle **901** attached to the proximal end **717** of the dilator **703.** The handle **901** can be axially-fixed relative to the dilator **703** such that movement of the handle **901** (e.g., along the dilator axis **715**) can cause corresponding movement of the dilator **703** along the dilator axis **715.** For example, in aspects, the handle **901** can be moved in a first movement direction **903** (e.g., a delivery direction) to move the dilator **703** further into the vessel **40.** Due to the handle **901** being axially-fixed, movement of the handle **901** in the first movement direction **903** can cause the dilator **703** to likewise move in the first movement direction **903.** Similarly, the handle **901** can be moved in a second movement direction **905** (e.g., a retraction direction) to retract the dilator **703** from the vessel **40.** Due to the handle **901** being axially-fixed, movement of the handle **901** in the second movement direction **905** can cause the dilator **703** to likewise move in the second movement direction **905.**

In aspects, the handle **901** can comprise a non-constant cross-sectional size along a length of the handle **901.** For example, the handle **901** can comprise a first peak portion **909** and a valley portion **911,** with the first peak portion **909** comprising a larger cross-sectional size than the valley portion **911.** The first peak portion **909** can be closer to the dilator **703** (e.g., the distal end **719,** for example) than the valley portion **911.** In this way, the first peak portion **909** can be located at an end of the handle **901.** In aspects, a second peak portion **913** can be located on an opposite side of the valley portion **911,** such that the valley portion **911** is located between the first peak portion **909** and the second peak portion **913.** The second peak portion **913** can comprise a larger cross-sectional size than the valley portion **911.** Accordingly, a physician can manipulate the handle **901,** for example, by holding the handle **901** at the valley portion **911.** The physician can move the handle **901,** and, thus, the dilator **703,** in the first movement direction **903** by applying a force to the first peak portion **909** in the first movement direction **903.** Alternatively, the physician can move the handle **901,** and, thus, the dilator **703,** in the second movement direction **905** by applying a force to the second peak portion **913** in the second movement direction **905.** In this way, the non-constant cross-sectional size of the handle **901** can facilitate gripping and manipulating of the handle and allow a movement force to be translated from the handle **901** to the dilator **703.** Accordingly, methods can comprise moving the dilator **703** axially by applying a force to the handle **901** that is attached adjacent to the proximal end **717** of the dilator **703,** with the handle **901** fixed (e.g., axially fixed) relative to the dilator **703** and comprising a non-constant cross-sectional size along a length of the handle **901.**

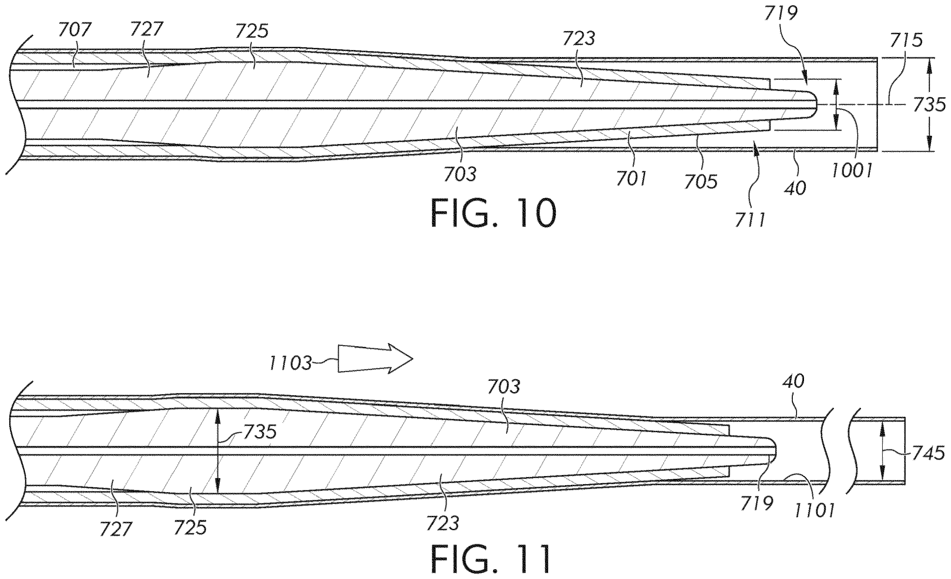

**FIG. 10** illustrates the dilator **703** received within the elongated chamber **707** of the sheath **701.** Methods can comprise positioning the sheath **701** within the vessel **40,** with the sheath **701** comprising the wall **705** surrounding the elongated chamber **707.** Methods can further comprise inserting the dilator **703** within the chamber **707.** The dilator **703** extends along the dilator axis **715** between the proximal end **717** and the distal end **719,** with the dilator **703** comprising the first tapered region **723** with an increasing diameter from the distal end **719,** the central region **725** coaxial with and in contact with the first tapered region **723** and comprising a substantially constant diameter, and the second tapered region **727** extending from and in contact with the central region **725.** The second tapered region **727** comprises a tapered shape with a decreasing diameter from the central region **725.** By receiving the dilator **703** within the sheath **701,** methods can comprise radially expanding (e.g., dilating) the sheath **701** by contacting the wall **705** with the central region **725.** For example, initially, the sheath **701** can comprise a sheath diameter **1001** that may be less than the second diameter **735** of the central region **725.** Upon being inserted into the elongated chamber **707** of the sheath **701,** the dilator **703** can cause the sheath **701** to radially expand. For example, the first tapered region **723** and the central region **725** can apply an outward radial force to the wall **705** of the sheath **701,** thus causing the sheath **701** to radially expand and increase in diameter. In aspects, when the sheath **701** and the dilator **703** are positioned within the vessel **40,** the vessel **40** can likewise radially expand. Accordingly, in the position illustrated in **FIG. 10****,** the central region **725** of the dilator **703** may contact the wall **705** of the sheath **701** and may not contact the vessel **40.**

**FIG. 11** illustrates the dilator **703** received within the vessel **40** such that the dilator **703** can contact a vessel wall **1101** of the vessel **40.** For example, methods can comprise radially expanding the vessel **40** by contacting the vessel wall **1101** with the central region **725.** For example, the dilator **703** can be moved in a movement direction **1103** such that the first tapered region **723** can move relative to the vessel wall **1101.** As the dilator **703** moves in the movement direction **1103,** the first tapered region **723** can apply an outward radial force to the vessel wall **1101.** Due to the elongated tapered shape of the first tapered region **723** in which the first tapered region **723** comprises a gradually increasing diameter from the distal end **719,** the vessel **40** may be radially-expanded in a gradual manner, thus reducing the risk of damage to the vessel **40.** The vessel diameter **745** may continue to increase at least until the vessel wall **1101** contacts the central region **725.** The central region **725** is the portion of the dilator **703** comprising a maximum diameter (e.g., the second diameter **735**). As such, the central region **725** can cause a maximum radial expansion of the vessel wall **1101** such that the vessel diameter **745** at the portion of the vessel **40** in contact with the central region **725** may be substantially equal to, or slightly larger than, the second diameter **735** of the central region **725.** The second tapered region **727** may comprise a decreasing cross-sectional size from the central region **725,** such that a portion of the second tapered region **727** may contact the vessel wall **1101.** In this way, the central region **725** is the portion of the dilator **703** that exerts a maximum radial force upon the vessel wall **1101,** while a lesser or negligible radial force may be exerted by the first tapered region **723,** the second tapered region **727,** and the proximal shaft region **729.** In this way, the total radial force exerted upon the vessel wall **1101** along the length of the dilator **703** may be reduced, since the dilator **703** comprises one region of maximum cross-sectional size (e.g., at the central region **725**) while other regions of the dilator **703** comprise a smaller cross-sectional size.

The dilator **703** can comprise multiple functions related to the vessel **40** and the sheath **701.** For example, due to the maximum diameter of the dilator **703** at the central region **725,** the dilator **703** can dilate and crack calcium in the vessel **40** (e.g., the arteriotomy and iliofemoral vessel, for example). In this way, access through iliofemoral vessels for large bore catheter devices is facilitated. Due to the relative short length of the central region **725,** the risk of iliac evulsion is reduced. In addition, when positioned in the sheath **701,** the dilator **703** can dilate the expandable sheath **701** to allow for access through the expandable sheath **701** for a large bore catheter device. Due to the size of the second diameter **735** (e.g., which can be about 1Fr to about 1.5Fr less than the device (e.g., valve, etc.) that will pass through the expandable sheath **701,** the dilator **703** does not induce excess vessel dilation of the vessel **40** since the vessel **40** is dilated below a max dilation that the device (e.g., valve, etc.) may impart when passing through the sheath **701** and/or the vessel **40.** The dilator **703** can comprise a material that may reduce contact surface area between the dilator **703** and one or more of the sheath **701** and/or the vessel **40.** For example, some or all of the dilator **703** can comprise one or more of a thermoplastic elastomer, a high-density polyethylene, a low-density polyethylene, a thermoplastic polyurethane, or polyamides. In addition, or in the alternative, some or all of the dilator **703** can be coated with a low-friction coating, such as a hydrophilic coating, for example, that can reduce friction and facilitate movement of the dilator **703.**

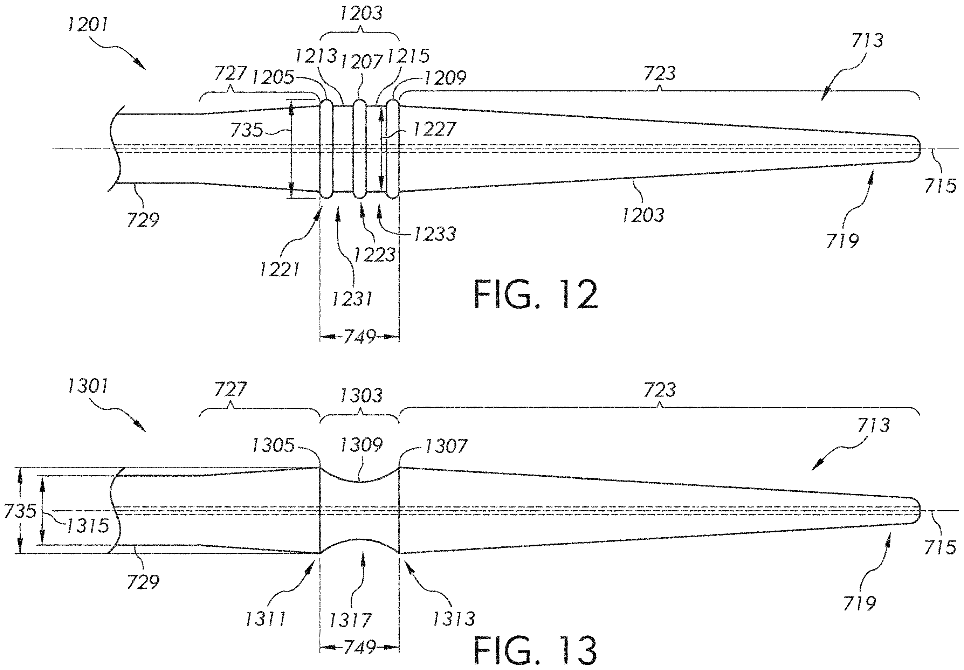

**FIG. 12** illustrates an additional embodiment of a dilator **1201** comprising the tapered end portion **713.** In aspects, the tapered end portion **713** of the dilator **1201** can comprise some similarities to the tapered end portion **713** of the dilator **703** illustrated in **FIGS. 7-11****.** For example, the first tapered region **723,** the second tapered region **727,** and the proximal shaft region **729** of the dilator **1201** may be substantially identical to the first tapered region **723,** the second tapered region **727,** and the proximal shaft region **729** of the dilator **703.** The dilator **1201** can comprise a central region **1203** that is positioned between the first tapered region **723** and the second tapered region **727** and comprises some similarities to the central region **725.** For example, the central region **1203** can comprise the second diameter **735** that is a maximum diameter of the central region **1203.** However, the diameter of the central region **1203** may be non-constant along the central length **749** of the central region **1203,** with the central region **1203** comprising one or more peak portions and one or more valley portions.

The central region **1203** can comprise a first peak portion **1205,** a second peak portion **1207,** and a third peak portion **1209,** and a first valley portion **1213** and a second valley portion **1215.** The peak portions **1205, 1207, 1209** comprise outcroppings, extensions, projections, protuberances, etc. that extend radially outwardly from the central region **1203** and extend circumferentially around an outer surface of the central region **1203.** In aspects, the peak portions **1205, 1207, 1209** can comprise a rounded shape (e.g., rounded along the dilator axis **715**) and comprise the second diameter **735.** In this way, the peak portions **1205, 1207, 1209** can comprise the maximum diameter of the central region **1203.** The peak portions **1205, 1207, 1209** may be substantially identical in size and shape, with the peak portions **1205, 1207, 1209** spaced apart along the dilator axis **715** and located at differing axial locations. For example, the first peak portion **1205** can be located at a first axial location **1221** that is at an end of the central region **1203** adjacent to the second tapered region **727.** The second peak portion **1207** can be located at a second axial location **1223** that is near the middle of the central region **1203,** with a distance separating the distal end **719** and the second axial location **1223** less than a distance separating the distal end **719** and the first axial location **1221.** In aspects, the third peak portion **1209** can be located at an end of the central region **1203** adjacent to the first tapered region **723** such that the first peak portion **1205** and the third peak portion **1209** are at opposing ends of the central region **1203.**

The valley portions **1213, 1215** can comprise substantially constant diameters between adjacent peak portions, for example, with the valley portions **1213,1215** comprising a valley diameter **1227** and extending circumferentially around an outer surface of the central region **1203.** In aspects, the valley diameter **1227** may be less than the second diameter **735** such that the valley portions **1213, 1215** comprise a minimum diameter of the central region **1203.** The valley portions **1213, 1215** can be spaced apart along the dilator axis **715** and located at differing axial locations. For example, the first valley portion **1213** can be located at a third axial location **1231** that is between the first axial location **1221** and the second axial location **1223.** The fourth axial location **1233** can be adjacent to the second axial location **1223** and on an opposite side of the second axial location **1223** from the third axial location **1231.** In this way, the first valley portion **1213** may be located axially between the first peak portion **1205** and the second peak portion **1207.** The second peak portion **1207** may be located axially between the first valley portion **1213** and the second valley portion **1215.** Accordingly, the first axial location **1221** and the second axial location **1223** can comprise the second diameter **735,** with the third axial location **1231** positioned between the first axial location **1221** and the second axial location **1223** and comprising the valley diameter **1227** that is less than the second diameter **735.** In this way, the dilator **1201** can function substantially identically to the dilator **703** illustrated in **FIGS. 7-11**, with the central region **1203** causing radial-expansion of the vessel **40** and/or the sheath **701.** Due to the central region **1203** comprising the non-constant diameter, the total radial force exerted upon the walls **705, 1101** along the length of the dilator **1201** may be reduced, such as, for example, by being limited to the peak portions **1205, 1207, 1209** that contact the walls **705, 1101.**

**FIG. 13** illustrates an additional embodiment of a dilator **1301** comprising the tapered end portion **713.** In aspects, the first tapered region **723,** the second tapered region **727,** and the proximal shaft region **729** of the dilator **1301** may be substantially identical to the first tapered region **723,** the second tapered region **727,** and the proximal shaft region **729** of the dilators **703, 1201.** The dilator **1301** can comprise a central region **1303** that is positioned between the first tapered region **723** and the second tapered region **727** and comprises some similarities to the central regions **725, 1203.** For example, the central region **1303** can comprise the second diameter **735** that is a maximum diameter of the central region **1303.** However, the diameter of the central region **1303** may be non-constant along the central length **749** of the central region **1203,** with the central region **1203** comprising one or more peak portions and one or more valley portions.

The central region **1303** can comprise a first peak portion **1305** and a second peak portion **1307,** and a valley portion **1309** positioned between the first peak portion **1305** and the second peak portion **1307.** The peak portions **1305, 1307** extend circumferentially around an outer surface of the central region **1303.** The peak portions **1305, 1307** can comprise the maximum diameter (e.g., the second diameter **735**) of the central region **1303.** For example, the peak portions **1305, 1307** may be substantially identical in size and shape, with the peak portions **1305, 1307** spaced apart along the dilator axis **715** and located at differing axial locations. For example, the first peak portion **1305** can be located at a first axial location **1311** that is at an end of the central region **1303** adjacent to the second tapered region **727.** The second peak portion **1307** can be located at a second axial location **1313** that is near an opposing end of the central region **1303** adjacent to the first tapered region **723,** with a distance separating the distal end **719** and the second axial location **1313** less than a distance separating the distal end **719** and the first axial location **1311.** In this way, the first peak portion **1305** and the second peak portion 1307 are at opposing ends of the central region **1303.**

The valley portion **1309** can comprise a non-constant diameter, for example, by comprising a valley diameter **1315** that is less than the second diameter **735.** In aspects, the valley portion **1309** can comprise a rounded shape with a decreasing diameter from the first peak portion **1305** toward a center of the valley portion **1309,** and a decreasing diameter from the second peak portion **1307** toward a center of the valley portion **1309.** In this way, the valley portion **1309** can comprise a minimum diameter (e.g., the valley diameter **1315**) at an axial center of the valley portion **1309** located substantially at a midpoint between the first peak portion **1305** and the second peak portion **1307.** The valley portion **1309** is located at a third axial location **1317** that is between the first axial location **1311** and the second axial location **1313.** In this way, the valley portion **1309** may be located axially between the first peak portion **1305** and the second peak portion **1307.** Accordingly, the first axial location **1311** and the second axial location **1313** can comprise the second diameter **735,** with the third axial location **1317** positioned between the first axial location **1311** and the second axial location **1313** and comprising the valley diameter **1315** that is less than the second diameter **735.** In this way, the dilator **1301** can function substantially identically to the dilators **703, 1201** illustrated in **FIGS. 7-12****,** with the central region **1303** causing radial-expansion of the vessel **40** and/or the sheath **701.** Due to the central region **1303** comprising the non-constant diameter, the total radial force exerted upon the walls **705, 1101** along the length of the dilator **1301** may be reduced, such as, for example, by being limited to the peak portions **1305, 1307** that contact the walls **705, 1101.**

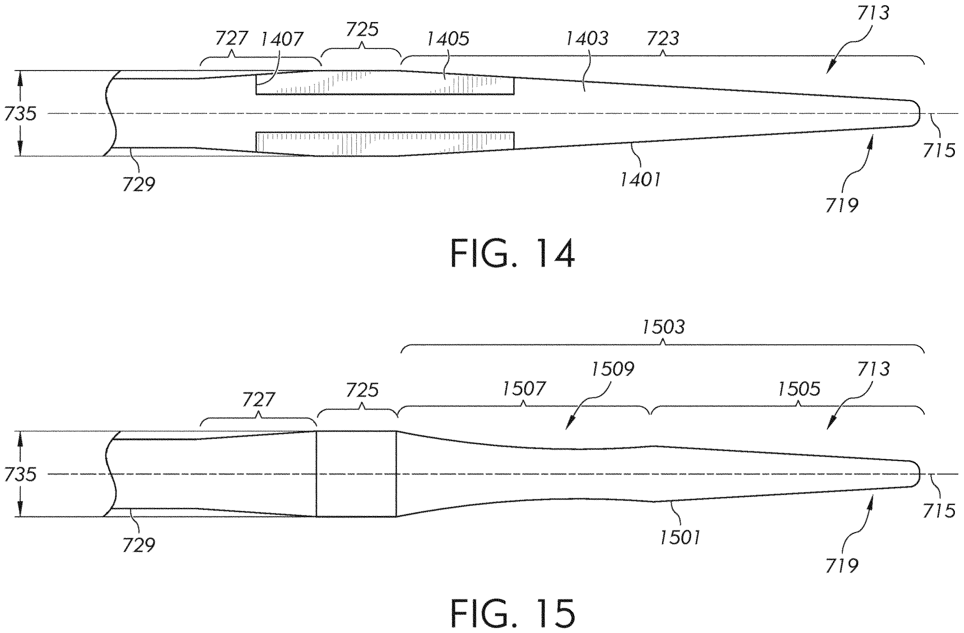

**FIG. 14** illustrates an additional embodiment of a dilator **1401** comprising the tapered end portion **713.** In aspects, the first tapered region **723,** the second tapered region **727,** the central region **725,** and the proximal shaft region **729** of the dilator **1401** may be substantially identical in shape and function to the first tapered region **723,** the second tapered region **727,** the central region **725,** and the proximal shaft region **729** of the dilator **703.** However, the dilator **1401** can comprise a plurality of materials, for example, with the central region **725** comprising a material that is different than a material of the proximal shaft region **729.** For example, the dilator **1401** can comprise a first material portion **1403** and a second material portion **1405.** The first material portion **1403** can comprise some or all of the first tapered region **723,** the second tapered region **727,** and the proximal shaft region **729.** The second material portion **1405** can comprise some or all of the central region **725,** the first tapered region **723,** and the second tapered region **727.** In aspects, the first material portion **1403** can comprise a different material than the second material portion **1405.** In aspects, the second material portion **1405** can be received within a recess **1407** defined within the first material portion **1403.** The first material portion **1403** can comprise a portion of the first tapered region **723** and a portion of the second tapered region **727.** The second material portion **1405** can comprise the central region **725,** a remaining portion of the first tapered region **723,** and a remaining portion of the second tapered region **727.** In aspects, the first material portion **1403** can comprise a softer and more flexible material than the second material portion **1405.** In this way, the first material portion **1403** can bend and/or flex as the dilator **1401** moves through the vessel **40,** while the second material portion **1405,** comprising the harder material, can apply the outward radial force and cause radial expansion of the vessel **40** and/or the sheath **701.**

**FIG. 15** illustrates additional embodiments of a dilator **1501** comprising the tapered end portion **713.** In aspects, the second tapered region **727,** the central region **725,** and the proximal shaft region **729** of the dilator **1501** may be substantially identical to the second tapered region **727,** the central region **725,** and the proximal shaft region **729** of the dilator **703.** However, in contrast to the first tapered region **723** of the dilator **703,** the dilator **1501** may comprise a first tapered region **1503** that increases in diameter from the distal end **719** to the first central end **731** at a non-constant rate. For example, the first tapered region **1503** can comprise a first tapered portion **1505** and a second tapered portion **1507.** The first tapered portion **1505** can extend a distance from the distal end **719** toward the first central end **731.** The first tapered portion **1505** may be substantially identical to a corresponding region of the first tapered region **723** of the dilator **703,** with the first tapered portion **1505** increasing in diameter at a constant rate from the distal end **719.** However, the second tapered portion **1507** can be positioned between the first tapered portion **1505** and the central region **725,** with the second tapered portion **1507** increasing in diameter at a non-constant rate from the first tapered portion **1505** to the central region **725.** For example, the second tapered portion **1507** can comprise a valley portion **1509** that is rounded in an axial direction along the dilator axis **715.** The second tapered portion **1507** can initially (e.g., from the first tapered portion **1505**) increase in diameter at a gradual rate (e.g., along a first half of the second tapered portion **1507**) followed by increasing in diameter at a faster rate (e.g., along a second half of the second tapered portion **1507**). In this way, the first tapered region **1503** can comprise one portion (e.g., the first tapered portion **1505**) that increases in diameter at a constant rate and another portion (e.g., the second tapered portion **1507**) that increases in diameter at a non-constant rate.

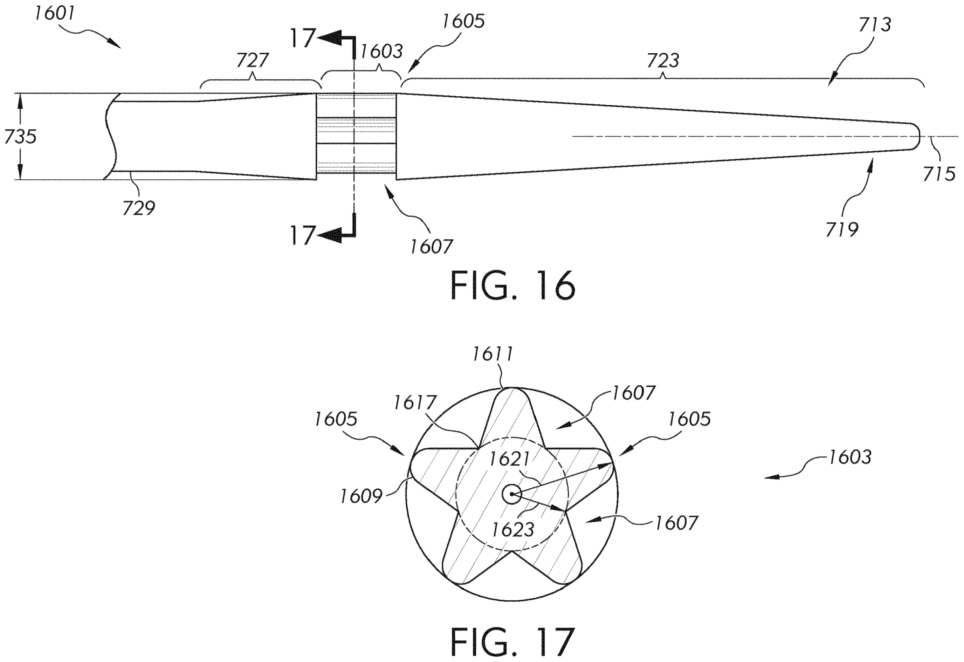

**FIGS. 16-17** illustrate additional embodiments of a dilator **1601** comprising the tapered end portion **713.** **FIG. 16** illustrates a side view of the tapered end portion **713** of the dilator **1601** and **FIG. 17** illustrates a cross-sectional view of **FIG. 16** along lines **17-17** of **FIG. 16****.** In aspects, the first tapered region **723,** the second tapered region **727** and the proximal shaft region **729** of the dilator **1601** may be substantially identical to the first tapered region **723,** the second tapered region **727** and the proximal shaft region 729 of the dilator **703.** However, in contrast to the central region **725** of the dilator **703,** the dilator **1601** may comprise a central region **1603** in which a cross-sectional size of the central region **1603** is non-constant about a circumferential perimeter of the central region **1603.**

Referring to **FIG. 17****,** the central region **1603** can comprise a plurality of peak portions **1605** and a plurality of valley portions **1607.** The plurality of peak portions **1605** can comprise a first peak portion **1609,** a second peak portion **1611,** etc., and the plurality of valley portions **1607** can comprise a first valley portion **1617,** etc. While the dilator 1601 in FIG. 17 is illustrated as comprising five peak portions and five valley portions, any number (e.g., one or more) of peak portions and/or valley portions may be provided. In aspects, the first valley portion **1617** may be located circumferentially between the first peak portion **1609** and the second peak portion **1611** about the circumferential perimeter of the central region **1603.** In this way, the central region **1603** can comprise alternating peak portions and valley portions, with a valley portion positioned between two peak portions, and a peak portion positioned between two valley portions. The plurality of peak portions **1605** can comprise a peak radius **1621** and the plurality of valley portions **1607** can comprise a valley radius **1623.** In aspects, the peak radius **1621** may be greater than the valley radius **1623,** such that the cross-sectional size, or radius, of the central region **1603** may be non-constant about the circumferential perimeter. In aspects, the peak radius **1621** may be about half of the second diameter **735** such that the central region **1603** can comprise a cross-sectional size that is substantially equal to the second diameter **735** of the central region **725.** In this way, the dilator **1601** can function substantially identically to the dilators **703, 1201, 1301, 1401, 1501** illustrated in **FIGS. 7-****15,** with the central region **1603** causing radial-expansion of the vessel **40** and/or the sheath **701.** Due to the central region **1603** comprising the non-constant cross-sectional size, the total radial force exerted upon the walls **705, 1101** about a perimeter of the central region 1603 may be reduced, such as, for example, by being limited to the plurality of peak portions **1605** that contact the walls **705, 1101.**

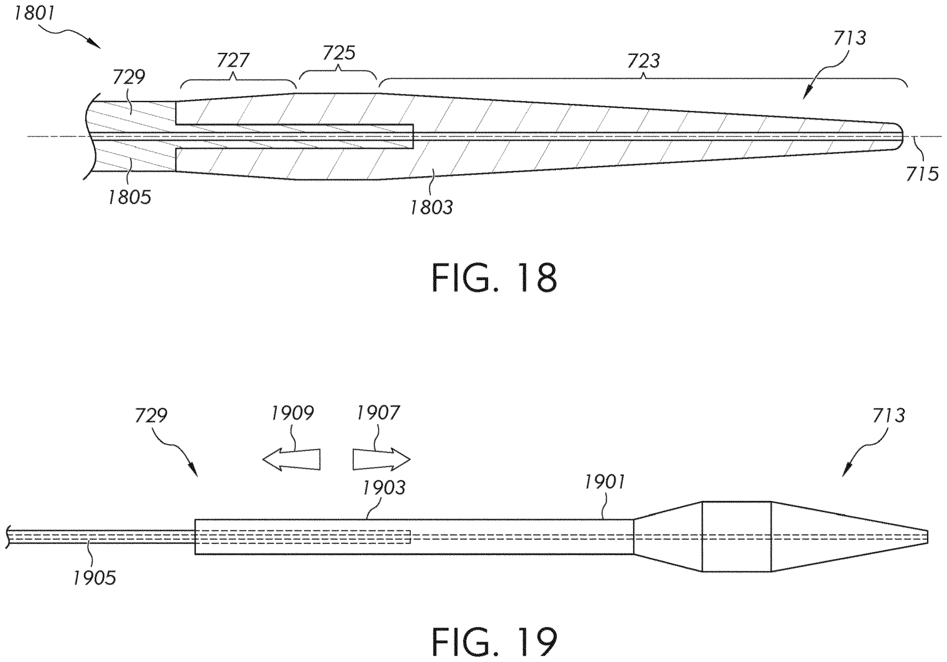

**FIG. 18** illustrates additional embodiments of a dilator **1801** comprising the tapered end portion **713.** In aspects, the first tapered region **723,** the second tapered region **727,** the central region **725,** and the proximal shaft region **729** of the dilator **1801** may be substantially identical in shape and function to the first tapered region **723,** the second tapered region **727,** the central region **725,** and the proximal shaft region **729** of the dilator **703.** However, the dilator **1801** can comprise a plurality of materials, for example, with the first tapered region **723,** the second tapered region **727,** and the central region **725** comprising a material that is different than a material of the proximal shaft region **729.** For example, the dilator **1801** can comprise a first material portion **1803** and a second material portion **1805.** The first material portion **1803** can comprise the first tapered region **723,** the second tapered region **727,** and the central region **725,** and the second material portion **1805** can comprise the proximal shaft region **729.** In aspects, the second material portion **1805** can comprise a softer and more flexible material than the first material portion **1803.** In this way, the second material portion **1805** can bend and/or flex as the dilator **1801** moves through the vessel **40,** while the first material portion **1803,** comprising the harder material, can apply the outward radial force and cause radial expansion of the vessel **40** and/or the sheath **701.** In aspects, the first material portion **1803** can be formed by multimaterial injection molding, for example, over-molding.

**FIG. 19** illustrates additional embodiments of a dilator **1901** comprising the tapered end portion **713.** In aspects, the first tapered region **723,** the second tapered region **727,** and the central region **725** of the dilator **1901** may be substantially identical to the first tapered region **723,** the second tapered region **727** and the central region **725** of the dilator **703.** However, the proximal shaft region **729** of the dilator **1901** can comprise a plurality of shaft portions, for example, a first shaft portion **1903** and a second shaft portion **1905.** In aspects, the at least one lumen **771** can extend through the first shaft portion **1903** and the second shaft portion **1905.** The first shaft portion **1903** can be attached to the second tapered region **727.** The second shaft portion **1905** can be received at least partially within the first shaft portion **1903,** for example, with a channel defined within the first shaft portion **1903.** In this way, the first shaft portion **1903** and the second shaft portion **1905** can extend substantially coaxially along the dilator axis **715.** However, in aspects, the first shaft portion **1903** can move relative to the second shaft portion **1905** in a first movement direction **1907** and/or in a second movement direction **1909.** By moving in the first movement direction **1907,** the first shaft portion **1903** can move away from the second shaft portion **1905** such that the dilator **1901** can elongate or increase in total length. The second shaft portion **1905** can remain within the channel of the first shaft portion **1903** such that the first shaft portion **1903** may not detach or separate from the second shaft portion **1905.** By moving in the second movement direction **1909,** the first shaft portion **1903** can move toward the second shaft portion **1905** such that the dilator **1901** can retract or decrease in total length. In this way, the length of the dilator **1901** can be adjusted, for example, by moving the first shaft portion **1903** relative to the second shaft portion **1905.** The shaft portions **1903, 1905** can be implemented with none, some, or all of the dilators **703, 1201, 1301, 1401, 1501, 1601, 1801** disclosed herein.

It should be understood that while various aspects have been described in detail relative to certain illustrative and specific examples thereof, the present disclosure should not be considered limited to such, as numerous modifications and combinations of the disclosed features are possible without departing from the scope of the following claims.

Further disclosed herein is the subject-matter of the following clauses:
Clause 1. A prosthetic valve delivery assembly comprising:
   a dilator extending along a dilator axis between a proximal end and a distal end, the dilator comprising:
   a first tapered region extending along the dilator axis between the distal end and a first central end, the first tapered region comprising a tapered shape with a first diameter at the distal end and a second diameter at the first central end, the second diameter greater than the first diameter;
   a central region coaxial with the first tapered region and attached to the first central end, the central region comprising the second diameter that is substantially constant along a central length of the central region;
   a second tapered region extending along the dilator axis between a second central end and a third central end, the second central end attached to the central region, the second tapered region comprising a tapered shape with the second diameter at the second central end and a third diameter at the third central end, the third diameter less than the second diameter; and
   a proximal shaft region extending from and in contact with the third central end, the proximal shaft region comprising the third diameter such that a difference between the second diameter and the third diameter is within a French gauge range from about 3Fr to about 5Fr.
Clause 2. The prosthetic valve delivery assembly of claim 1, wherein the central region comprises a central length that is within a range from about 1 mm to about 150 mm.
Clause 3. The prosthetic valve delivery assembly of clause 1 or of any preceding clause, wherein the tapered shape of the second tapered region comprises a taper angle that is within a range from about 1 degree to about 30 degrees.
Clause 4. The prosthetic valve delivery assembly of clause 1 or of any preceding clause, wherein the second diameter is less than about 1.5 times a vessel diameter of the vessel.
Clause 5. The prosthetic valve delivery assembly of clause 1 or of any preceding clause, further comprising a handle attached adjacent to the proximal end of the dilator, the handle axially fixed relative to the dilator and comprising a non-constant cross-sectional size along a length of the handle.
Clause 6. The prosthetic valve delivery assembly of clause 1 or of any preceding clause, wherein the dilator comprises at least one lumen extending axially through the dilator.
Clause 7. The prosthetic valve delivery assembly of clause 6, wherein the at least one lumen comprises a first lumen, a second lumen, and a third lumen, the first lumen laterally offset from, and between, the second lumen and the third lumen.
Clause 8. The prosthetic valve delivery assembly of clause 1 or of any preceding clause, wherein the dilator comprises a plurality of materials.
Clause 9. A prosthetic valve delivery assembly comprising:
   a sheath comprising a wall surrounding an elongated chamber, the sheath configured to be received within a vessel; and
   a dilator configured to be received within the chamber and extending along a dilator axis between a proximal end and a distal end, the dilator comprising:
      a first tapered region extending along the dilator axis between the distal end and a first central end, the first tapered region comprising a tapered shape with a first diameter at the distal end and a second diameter at the first central end, the second diameter greater than the first diameter;
      a central region coaxial with the first tapered region and attached to the first central end, the central region comprising the second diameter that is less than about 1.5 times a vessel diameter of the vessel, the central region comprising a central length that is within a range from about 1 mm to about 150 mm;
      a second tapered region extending along the dilator axis between a second central end and a third central end, the second central end attached to the central region, the second tapered region comprising a tapered shape with the second diameter at the second central end and a third diameter at the third central end, the third diameter less than the second diameter, the tapered shape of the second tapered region comprising a taper angle that is within a range from about 1 degree to about 30 degrees; and
      a proximal shaft region extending from and in contact with the third central end, the proximal shaft region comprising the third diameter, the third diameter within a range from about 60% to about 90% of the second diameter.
Clause 10. The prosthetic valve delivery assembly of clause 9, wherein a diameter of the central region is non-constant along the central length of the central region, and the second diameter is a maximum diameter of the central region.
Clause 11. The prosthetic valve delivery assembly of clause 9 or 10, wherein the central region comprises a first axial location and a second axial location comprising the second diameter, and a third axial location is positioned between the first axial location and the second axial location and comprises a diameter less than the second diameter.
Clause 12. The prosthetic valve delivery assembly of clause 9 or of any one of clauses 9 to 11, wherein a cross-sectional size of the central region is non-constant about a circumferential perimeter of the central region.
Clause 13. The prosthetic valve delivery assembly of clause 9 or of any one of clauses 9 to 12, wherein the second diameter is constant along the central length of the central region.
Clause 14. The prosthetic valve delivery assembly of clause 9 or of any one of clauses 9 to 13, wherein the central region comprises a material that is different than a material of the proximal shaft region.
Clause 15. A method of expanding a sheath comprising:
   positioning a sheath within a vessel, the sheath comprising a wall surrounding an elongated chamber;
   inserting a dilator within the chamber, the dilator extending along a dilator axis between a proximal end and a distal end, the dilator comprising a first tapered region with an increasing diameter from the distal end, a central region coaxial with and in contact with the first tapered region and comprising a substantially constant diameter, and a second tapered region extending from and in contact with the central region, the second tapered region comprising a tapered shape with a decreasing diameter from the central region; and
   radially expanding the sheath by contacting the wall with the central region.
Clause 16. The method of clause 15, wherein the central region comprises a central length that is within a range from about 1 mm to about 150 mm.
Clause 17. The method of clause 15, further comprising moving the dilator axially by applying a force to a handle that is attached adjacent to the proximal end of the dilator, the handle fixed relative to the dilator and comprising a non-constant cross-sectional size along a length of the handle.
Clause 18. The method of clause 15, further comprising receiving a first guidewire within a first lumen that extends axially through the dilator.
Clause 19. The method of clause 18, further comprising receiving a second guidewire within a second lumen that extends axially through the dilator, and a third guidewire within a third lumen that extends axially through the dilator, the first lumen laterally offset from, and between, the second lumen and the third lumen.
Clause 20. The method of clause 15, further comprising radially expanding the vessel by contacting a vessel wall of the vessel with the central region.

Further disclosed herein is a prosthetic valve delivery assembly. The prosthetic valve delivery assembly includes a dilator including a first tapered region extending along a dilator axis. The first tapered region includes a tapered shape with a first diameter at a distal end and a second diameter at a first central end. The second diameter is greater than the first diameter. A central region includes the second diameter that is substantially constant along a central length of the central region. A second tapered region includes a tapered shape with the second diameter at the second central end and a third diameter at a third central end. The third diameter is less than the second diameter. A proximal shaft region extends from the third central end. The proximal shaft region includes the third diameter such that a difference between the second diameter and the third diameter is within a French gauge range from about 3Fr to about 5Fr.

## Claims

1. A prosthetic valve delivery assembly comprising:
a dilator extending along a dilator axis between a proximal end and a distal end, the dilator comprising:
a first tapered region extending along the dilator axis between the distal end and a first central end, the first tapered region comprising a tapered shape with a first diameter at the distal end and a second diameter at the first central end, the second diameter greater than the first diameter;
a central region coaxial with the first tapered region and attached to the first central end, the central region comprising the second diameter that is substantially constant along a central length of the central region;
a second tapered region extending along the dilator axis between a second central end and a third central end, the second central end attached to the central region, the second tapered region comprising a tapered shape with the second diameter at the second central end and a third diameter at the third central end, the third diameter less than the second diameter; and
a proximal shaft region extending from and in contact with the third central end, the proximal shaft region comprising the third diameter such that a difference between the second diameter and the third diameter is within a French gauge range from about 3Fr to about 5Fr.

2. The prosthetic valve delivery assembly of claim 1, wherein the central region comprises a central length that is within a range from about 1 mm to about 150 mm.

3. The prosthetic valve delivery assembly of any preceding claim, wherein the tapered shape of the second tapered region comprises a taper angle that is within a range from about 1 degree to about 30 degrees.

4. The prosthetic valve delivery assembly of any preceding claim, wherein the second diameter is less than about 1.5 times a vessel diameter of the vessel.

5. The prosthetic valve delivery assembly of any preceding claim, further comprising a handle attached adjacent to the proximal end of the dilator, the handle axially fixed relative to the dilator and comprising a non-constant cross-sectional size along a length of the handle.

6. The prosthetic valve delivery assembly of any preceding claim, wherein the dilator comprises at least one lumen extending axially through the dilator.

7. The prosthetic valve delivery assembly of claim 6, wherein the at least one lumen comprises a first lumen, a second lumen, and a third lumen, the first lumen laterally offset from, and between, the second lumen and the third lumen.

8. The prosthetic valve delivery assembly of any preceding claim, wherein the dilator comprises a plurality of materials.

9. A prosthetic valve delivery assembly comprising:
a sheath comprising a wall surrounding an elongated chamber, the sheath configured to be received within a vessel; and
a dilator configured to be received within the chamber and extending along a dilator axis between a proximal end and a distal end, the dilator comprising:
a first tapered region extending along the dilator axis between the distal end and a first central end, the first tapered region comprising a tapered shape with a first diameter at the distal end and a second diameter at the first central end, the second diameter greater than the first diameter;
a central region coaxial with the first tapered region and attached to the first central end, the central region comprising the second diameter that is less than about 1.5 times a vessel diameter of the vessel, the central region comprising a central length that is within a range from about 1 mm to about 150 mm;
a second tapered region extending along the dilator axis between a second central end and a third central end, the second central end attached to the central region, the second tapered region comprising a tapered shape with the second diameter at the second central end and a third diameter at the third central end, the third diameter less than the second diameter, the tapered shape of the second tapered region comprising a taper angle that is within a range from about 1 degree to about 30 degrees; and
a proximal shaft region extending from and in contact with the third central end, the proximal shaft region comprising the third diameter, the third diameter within a range from about 60% to about 90% of the second diameter.

10. The prosthetic valve delivery assembly of claim 9, wherein a diameter of the central region is non-constant along the central length of the central region, and the second diameter is a maximum diameter of the central region.

11. The prosthetic valve delivery assembly of claim 9 or 10, wherein the central region comprises a first axial location and a second axial location comprising the second diameter, and a third axial location is positioned between the first axial location and the second axial location and comprises a diameter less than the second diameter.

12. The prosthetic valve delivery assembly of any one of claims 9 to 11, wherein a cross-sectional size of the central region is non-constant about a circumferential perimeter of the central region.

13. The prosthetic valve delivery assembly of any one of claims 9 to 12, wherein the second diameter is constant along the central length of the central region.

14. The prosthetic valve delivery assembly of any one of claims 9 to 13, wherein the central region comprises a material that is different than a material of the proximal shaft region.
